# EUROPEAN PATENT APPLICATION

(11) **EP 3 893 244 A2**
(43) Date of publication of application: **13.10.2021**
(21) Application number: 21182004.8
(22) Date of filing: 28.06.2021
(51) Int. Cl.: G16H 20/13

(54) **A METHOD OF PROVIDING MEDICINE INFORMATION**

(71) Applicant: Abbott Products Operations AG, 4123 Allschwil (CH)
(72) Inventor: SUERIG, Ulf, 4123 Allschwil (CH); HUTT, Douglas, 4123 Allschwil (CH); BRUIL, Menno, 7207 RT Zutphen (NL)
(74) Representative: Sadler, Peter Frederick

(57) **Abstract**

The present disclosure provides a method of providing medicine information to a patient is provided. The method comprises: scanning, at a mobile terminal associated with the patient, a code disposed upon packaging for a medication; determining, by the mobile terminal, an identification associated with the medication based upon the scanned code; sending, from the mobile terminal to a server, the determined identification; receiving, at the mobile terminal from the server, medicine information associated with the identification; and presenting, at the mobile terminal, the medicine information to the patient. A system and devices for use with the method are also disclosed.

## Description

### TECHNICAL FIELD

The present disclosure relates to a method of, and a system and devices for, providing medicine information to a patient. More particularly, it relates to a method for scanning a code provided on medication packaging to present medicine information relating to the medication in the medication packaging to a patient, and a system and devices for use therewith.

### BACKGROUND

Patients often take, or are prescribed by a doctor or other care giver, medication to take as part of treatment for numerous conditions. Traditionally, prescription medication is obtained at a pharmacy and over-the-counter medication may be bought by a patient at a pharmacy or elsewhere, such as a supermarket. The medication is bought in a packet, which also includes medicine information in the form of a Patient Information Leaflet (PIL). The provision of a PIL with medication is a legal requirement in many countries. The PIL is provided in the form of a paper leaflet which is folded and placed inside the medication packaging, and comprises information such as who should or should not take the medication, known side effects of the medication, and other relevant information regarding the medication.

However, such traditional methods of providing medicine information to a patient have a number of drawbacks. One disadvantage is that a paper PIL can easily be lost, damaged or destroyed inadvertently by a patient. Another disadvantage is that the PIL cannot be customised to the patient, and so, for example, cannot inform them of their dosage regime which may be unique to them as prescribed by their doctor or other care giver. Similarly, if a patient is illiterate, or is abroad in a foreign country that they do not speak the language of, they may be unable to read the information provided on a PIL. Yet another disadvantage of traditional methods of providing medicine information is that a PIL is typically provided on a per-packet basis, yet medicine may be distributed on a per-pill (for example) basis meaning that a PIL cannot be readily provided to each patient. It would further be impractical to provide a number of leaflets equal to the number of pills (or other measures of medicine dosage) in a packet as this would make the packet prohibitively large, and be overly wasteful and inconvenient for many patients who purchase the entire packet of medication themselves and only need a single PIL. In addition to the above, a further disadvantage of paper leaflets and traditional PILs is that they are easy to counterfeit and it is not easy for a patient to be confident that they have bought genuine medicine.

Accordingly, it would be advantageous to provide a method of providing a patient with medicine information that overcomes some or all of these disadvantages. It would also be advantageous to provide a reliable method for verifying/authenticating a pharmaceutical product.

### SUMMARY OF INVENTION

The invention is defined in the independent claims, to which the reader is now directed. Preferred or advantageous features are set out in the dependent claims.

According to a first aspect of the invention, a method of providing medicine information to a patient is provided. The method comprises: scanning, at a mobile terminal associated with the patient, a code disposed upon packaging for a medication; determining, by the mobile terminal, an identification associated with the medication based upon the scanned code; sending, from the mobile terminal to a server, the determined identification; receiving, at the mobile terminal from the server, medicine information associated with the identification; and presenting, at the mobile terminal, the medicine information to the patient.

Compared to traditional methods of distributing medicine information to a patient, such as via paper Patient Information Leaflets (PILs), information presented to the user in accordance with the present invention can be kept up to date regardless of when the medicine is manufactured and sold. Additionally, information can readily be tailored to the patient. This include the language in which the information is presented to the patient, which means that regardless of where the medicine is bought, the information can be provided in a language that the patient can comprehend. Furthermore, the information cannot be accidentally lost or misplaced, as with a physical leaflet, and the patient can easily access the information at any point by scanning the medication packaging.

Optionally, the medicine information comprises a Patient Information Leaflet (PIL).

By utilising such an electronic Patient Information Leaflet (e-PIL), waste can be reduced since there is no need for paper leaflets. Furthermore, it means that a patient cannot lose the PIL, and that the PIL can be kept up to date.

Optionally, the method further comprises the step of authenticating, by the server, the identification; wherein if the server is able to authenticate the identification then the medicine information comprises an indicator that the identification was authenticated, and/or if the server is not able to authenticate the identification then the medicine information comprises an indicator that the identification was not authenticated.

By authenticating the medicine in this manner, the consumer can be confident that the medicine they are sold is not counterfeit medication, because such counterfeit medication would not have an identification that the server could authenticate.

Optionally, the method further comprises the step of determining, by the server, if the server has previously authenticated the identification. If the server is able to authenticate the identification but has previously authenticated the same identification then the medicine information comprises an indicator that the medicine may not be authentic.

This prevents the re-use of old, authentic packaging with counterfeit medication by counterfeiters by having each code as a "single use" code, which in turn further enhances the confidence that the patient can have that their medicine is genuine.

Optionally, the method further comprises the steps of: querying, by the server, a database to obtain a distribution history of the medication based on the identification; and receiving, at the server from the database, the distribution history of the medication based on the identification; wherein if the server is able to authenticate the identification then the medicine information further comprises a distribution history of the medication.

Presenting the patient with the distribution history of their medication can reassure them that the product is genuine and enable them to spot counterfeit medications, as well as help them to identify if the medication they purchased may be genuine but may have been stolen.

Optionally, prior to sending the determined identification to the server, the method further comprises, at the mobile terminal, determining the location of the mobile terminal; wherein the step of sending the determined identification further comprises sending, to the server, the location of the mobile terminal; and wherein the medicine information received from the server is at least in part based upon the location of the mobile terminal.

For example, the medicine information can be presented in a different language depending upon the location of the mobile terminal. Additionally, different jurisdictions may require different information be presented about the medicine, and so by basing the medicine information on the location of the mobile terminal, different information can be presented according to the relevant laws in different jurisdictions.

Optionally, the location of the mobile terminal can be determined through the use of a location function of the mobile terminal that actively determines location such as GNSS (e.g. GPS), WiFi or a mobile network. Alternatively location may be determined by data input such as patient input through the mobile terminal.

Optionally, the method further comprises the steps of, at the mobile terminal: receiving prescription information, the prescription information specifying one or more times at which the patient should take the medication and a dosage of the medication to be taken at the one or more times; receiving patient confirmation when the patient has taken a dose of the medication; and determining patient compliance with the prescription information.

By monitoring patient compliance, which can then be displayed, the patient can see whether they have been correctly taking their medication and can also show this information to their doctor or other care giver to enable them to provide better care, and removes the uncertainty associated with a patient having to remember how frequently they took their medication.

Optionally, the method further comprises the step of sending, from the mobile terminal to a patient's care provider, information relating to the determined patient compliance.

Automatically forwarding information about the patient compliance to the doctor or care giver means that the doctor or caregiver is better able to diagnose and care for the patient. This can help overcome issues such as patients being unwilling to indicate poor compliance or overestimating their compliance. In particular, such information can be useful in enabling the doctor or care giver to determine whether a course of medication is ineffective meaning a different treatment should be tried, or whether the problem is that the patient is failing to follow their prescribed treatment.

Optionally, the medicine information comprises an initial amount of medication associated with the identification, and the method further comprises the steps of, at the mobile terminal: updating the amount of medication associated with the identification by decreasing the amount based upon the prescription information and/or determined patient compliance; and notifying the patient when the updated amount of medication passes a predetermined threshold that indicates that the medication has almost been consumed.

Automatically monitoring how much medication the patient has used and how much remains means that the patient can easily see how much medication they have remaining, and be alerted when they are running low on medication. This means that they can manage the amount of medication they have and can buy or reorder their medication or prescription in a timely manner to avoid running out.

Optionally, the method further comprises the step of automatically ordering new medication by the mobile terminal when the updated amount of medication passes the predetermined threshold.

Automatically ordering new medication ensures that the patient does not run out of medication. As such, they can consistently take their medication and easily follow their plan of treatment, improving patient outcomes. This feature may be implemented in addition to, or as an alternative to, alerting the patient when they are running low on medication.

Optionally, the medicine information further comprises a link to a page of an online pharmacy through which the patient can order new medication. In particular the link is a computer actionable link such as a hyperlink and may be in the form of an HTML link or similar.

By including a link to an online pharmacy in the medication, through scanning the medication packet the patient can be taken to an online pharmacy to re-order their medicine. This makes it easy for the patient to top up their medication and ensure that they do not run out. As such, they can consistently take their medication and easily follow their plan of treatment, improving patient outcomes.

Optionally, the amount of medication is a number of doses, a volume of fluid or a number of pills or capsules. The amount of medication may include any unit or way of measuring a dose medication to be taken.

Optionally, the prescription information is received from a terminal associated with a health care provider.

Optionally the healthcare provider is one of a doctor, a pharmacist, or the prescriber of the medication.

Receiving the prescription information from a terminal associated with a doctor or other health care provider means that the patient can be sure that it is the correct information and that they have not misheard or misremembered the information, or have otherwise input the prescription information into their terminal incorrectly themselves. Furthermore, where the patient and doctor have difficulty communicating, such as may be the case if a patient is in a foreign country and they and the care giver speak different languages, the doctor or other health care provider can input the prescription information in the doctor's native language and this can be presented to the patient in the patient's native language according to the patient's preferred language settings on their terminal, overcoming this barrier.

Optionally, the method further comprises the step of outputting a notification to alert the patient when the prescription information indicates that the patient is due to take some or all of the medication.

Outputting such a notification ensures that the patient knows when to take the medication and means the patient is less likely to forget to take their medication, improving compliance and hence patient outcomes.

Optionally, the notification to alert the patient when the prescription information indicates that the patient is due to take some or all of the medication is output at a time based on at least the prescription information and user input from the patient.

Outputting the notification based on at least the prescription information and user input from the patient means that the patient can customise the notifications such that they are presented in a manner that suits them. In particular, it means that the patient can select the most appropriate reminder time for them to enable them to maximise compliance.

Optionally, the code is associated with an individual pill or capsule.

Having the code associated with individual pills or capsules of medication means that each individual pill or capsule can be authenticated. This overcomes the problems associated with PIL distribution when medication is distributed as individual pills or other irregular amounts not catered for by a complete packet of medication.

Optionally, the code is associated with a packet comprising multiple individual pills or capsules, and wherein the code can further identify each constituent pill or capsule in the packet.

Such code aggregation allows individual pills or capsules of medication to be easily tracked without scanning every individual pill when unnecessary, by allowing the whole packet to be scanned instead when it is not necessary to individually identify a pill or capsule.

Optionally, the code is one of a barcode, a QR code, or a GS1 2D DataMatrix.

Such codes can be easily and clearly printed on medication packaging, and can be reliably read by a number of devices. Advantageously, the code can be scanned using a camera or other component of a mobile telephone or similar user terminal, meaning that it is easy for any patient with access to a mobile phone to scan the code.

Optionally, the identification is a Global Trade Number.

Using a Global Trade Number is advantageous as such numbers are already required to be associated with medication and so the method can be implemented digitally without any physical change to packaging and distribution systems.

Optionally, at least a part of the medicine information is presented audibly or graphically.

Presenting some or all of the medicine information audibly or graphically can help to overcome a lack of patient literacy. In combination with the scanning of a code, this means that no literacy is required from patient and so ensures a patient is presented with correct information which can be kept up to date regardless of patient literacy.

Optionally, at least a part of the medicine information is presented through a digital avatar.

The use of a digital avatar means that the patient can be presented with a friendly interface which encourages patient compliance. The use of a digital avatar further aids comprehension of spoken word or audible medicine information to overcome a lack of patient literacy.

Optionally, the medicine information comprises holistic medical information and/or advice which may include information and/or advice on any of the following topics: diet, exercise, disease management, and how to take the medication.

Providing such holistic information based upon the scanned code means that information relevant to the patient can be provided based upon their condition/the medication they are taking which can help a patient correctly complete their course of treatment and provide further information that the patient can use to help improve patient outcomes.

According to a second aspect of the invention, a system comprising a mobile terminal and a server is provided. The system is configured to perform the method of the first aspect of the invention.

According to a third aspect of the invention, a mobile terminal is provided. The mobile terminal is configured for use in the system of the second aspect of the invention.

According to a fourth aspect of the invention, a server is provided. The server is configured for use in the system of the second aspect of the invention.

### BRIEF DESCRIPTION OF THE DRAWINGS

Non-limiting embodiments of the invention will be described in relation to the following figures.
Figure 1 illustrates a method according to the invention.
Figure 2 illustrates a mobile terminal displaying a page of an application for use with the invention.
Figure 3 illustrates a mobile terminal displaying a further page of an application for use with the invention.
Figure 4 illustrates a mobile terminal displaying a further page of an application for use with the invention.
Figure 5 illustrates a mobile terminal displaying a further page of an application for use with the invention.
Figure 6 illustrates a medication packet with which the invention can be used.
Figure 7 illustrates a blister pack of medication with which the invention can be used.
Figure 8 illustrates a system for use with the invention.

### DETAILED DESCRIPTION OF THE INVENTION

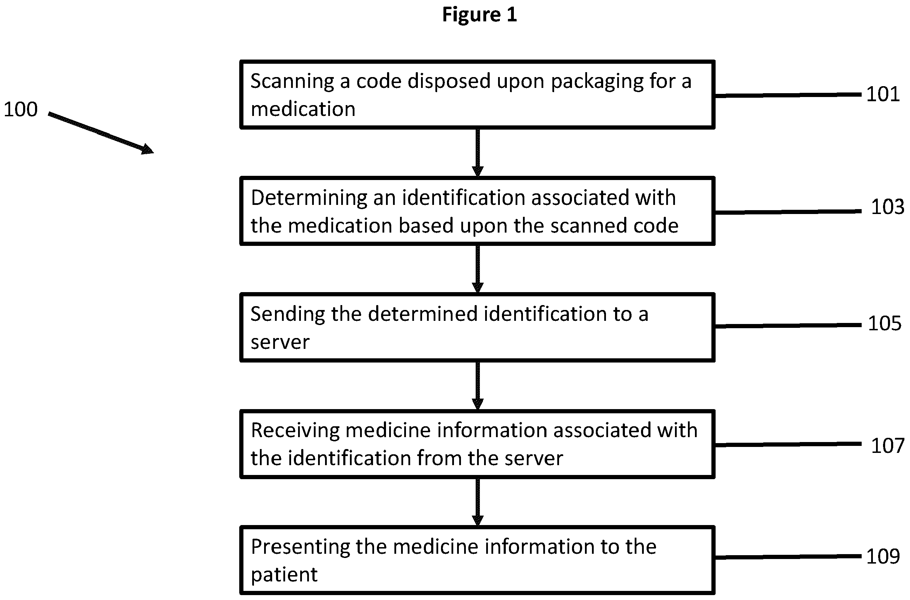

Figure 1 illustrates a method 100 for providing medicine information to a patient. In the illustrated method 100, all of the steps are performed at a mobile terminal associated with the patient to whom the medicine information is to be provided. Types of mobile terminal that the method could be used with include, but are not limited to, mobile phones, tablet devices, laptop computers, smart watches, and other similar devices. The functionality of a terminal to provide the methods of the invention may be provided through an application, or app, downloaded onto a mobile terminal or accessible through a web browser application or other network means.

Step 101 of the method 100 involves scanning a code disposed upon packaging for a medication. In this step, the patient uses their mobile terminal to scan a code that is disposed on the packaging of the medication they have bought and/or been prescribed. The code may be scanned using a camera of the mobile terminal.

Step 103 of the method 100 involves determining an identification associated with the medication based upon the scanned code. For example, the code may encode a serial number or other such form of identification. This identification is uniquely associated with the medication in the packaging.

Step 105 of the method 100 involves sending the determined identification to a server. In this step, the identification (e.g. a serial number) is sent from the mobile terminal to a server. The identification may be send in encoded and/or encrypted form. The server may be a dedicated and secure server that is managed by the manufacturer or distributor of the medicine with which the identification is associated.

Step 107 of the method involves receiving medicine information associated with the identification from the server. The server may use the identification to obtain the associated medicine information, for example by querying a database containing a correspondence between the identification and the medicine information. In some embodiments, the medicine information may comprise an electronic Patient Information Leaflet (an electronic PIL or e-PIL) that is associated with the medication associated with the identification.

Step 109 of the method involves presenting the medicine information to the patient. This could be in the form of text presented on a screen of the mobile terminal, or in another way, such as graphically or audibly, or in some combination thereof.

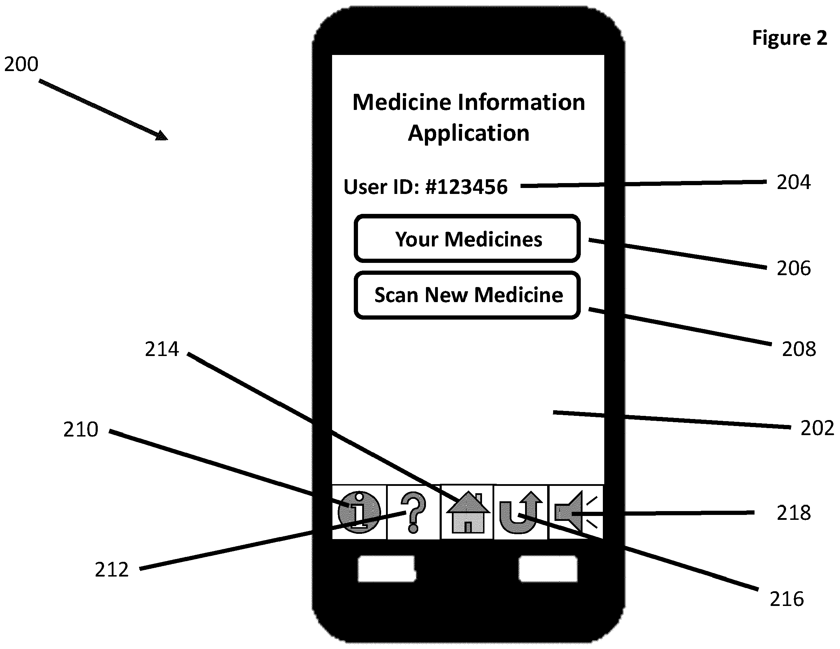

Figure 2 shows a mobile terminal 200, in this embodiment a mobile phone, configured to perform methods of the invention through the use of an application on the mobile phone. The mobile terminal 200 comprises a display 202. In this embodiment, a patient is associated with a user account of the application, as illustrated by user ID 204. In such an embodiment, the user may need to verify their identity before opening the application on the mobile terminal, for example through inputting a password, PIN, using fingerprint or facial recognition technologies, or any other suitable method known in the art. A user account is not necessary in some embodiments of the invention, in particular it is not needed if the medicine information presented to the user upon the scanning of a medicine packet comprises no personalised information (such as prescription information). However, for some features a user account may be preferred, such as if the medicine information includes personalised information, such as prescription information. It is noted that even if personalised information, such as prescription information, is to be provided in the medicine information, personal information is not necessarily stored. For example, a user may have a random account ID number which can be used to provide them with personalised information but does not itself contain any personal data.

In some embodiments, an application configured to scan a code and receive medicine information in accordance with the present invention may not have an account associated with it and may not store any personal data. It may, however, communicate with a second, separate application on the mobile terminal which does have a user account and does store personal information. The application may forward the medicine information received from a server to this second application, which can then integrate the received medicine information into the rest of the patient's digital healthcare environment.

Figure 2 also illustrates a number of application buttons 210-218. In the illustrated example these include one or more of an information button 210, a help button 212, a home button 214, a back or return button 216, and an audio button 218. The information button 210, when pressed, may provide the user with further information about the application or further information about an item currently being displayed on display 202. The help button 212, when pressed, may activate a help feature to provide assistance to a user in using the application. For example, it may result in the display a list of frequently asked questions and associated answers, or provide the user with contact information through which the user can seek further assistance. The home button 214, when pressed, may return the user to a specific page of the application, for example a home page. The back, or return, button 214, when pressed, may return a user to a page that the user was previously viewing. The audio button 218, when pressed, may describe or read aloud the content currently being presented on display 202. The application buttons 210-218 may be present on display 202 regardless of the current page of the application that the user is viewing. Alternatively, they may vary depending upon the content being displayed. It will also be noted that these buttons and functions are exemplary only, and that other buttons may be present, or no buttons at all, and that they may be presented to the user in any manner or arrangement on display 202.

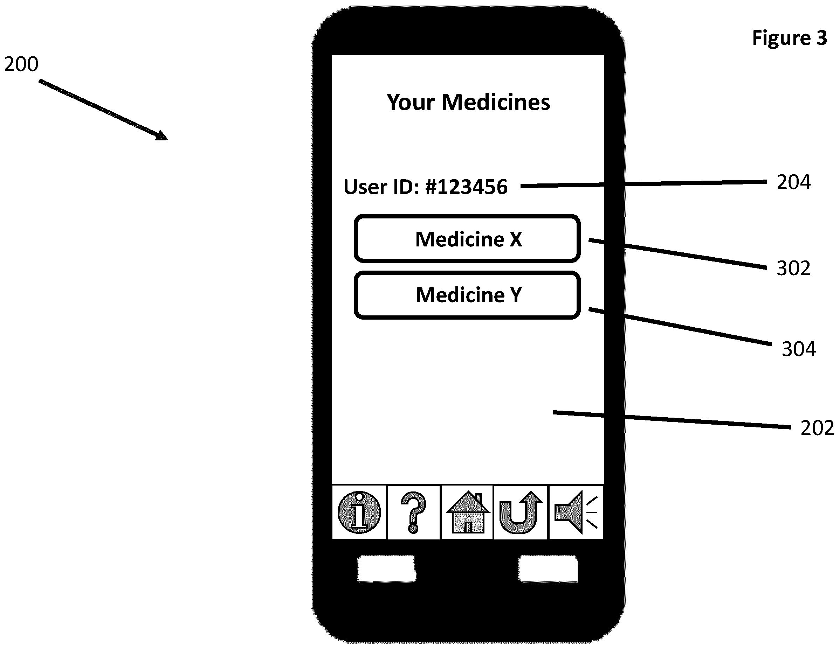

In Figure 2, a home page of a "Medicine Information Application" is shown, and the user is presented with a "Your Medicines" button 206 and a "Scan New Medicine" button 208. The "Your Medicines" button 206 takes the user to a page, or otherwise displays, a list of any previously scanned medications. An exemplary application page is shown in Figure 3. This may allow a user to see medicine information for medicines that they have previously scanned with the mobile terminal and which has previously been received from a server. As can be seen in Figure 3, the user has previously scanned two medicines, "Medicine X" and "Medicine Y". On display 202, a "Medicine X" button 302 is provided which will present the user with medicine information relating to "Medicine X" and a "Medicine Y" button 304 is provided which will present the user with medicine information relating to "Medicine Y".

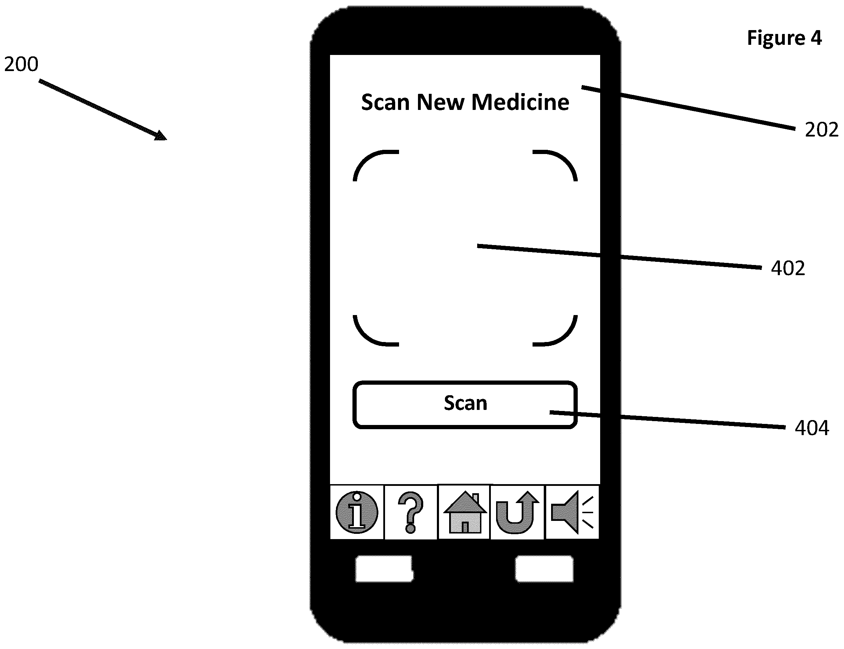

Returning to Figure 2, the "Scan New Medicine" button 208 takes the user to a page, or otherwise provides a means, for scanning a code on medication packaging. An exemplary application page is shown in Figure 4. In Figure 4, on display 202 the user is presented with a scanning region 402 and a "Scan" button 404. Whilst on this page, the application may communicate with a camera of the mobile terminal 200 such that a live image from the camera is presented in scanning region 402. By arranging the mobile terminal 200 and the packaging of medication to be scanned, the user can bring the code disposed on the packaging of the medicine into the scanning region 402. When the code is located in the scanning region 402, the user may press the "Scan" button 404 causing the mobile terminal to scan the code. Alternatively, in other embodiments, the code may be scanned automatically once the mobile terminal detects a code, e.g. when a code is detected within the scanning region 402, without further user input.

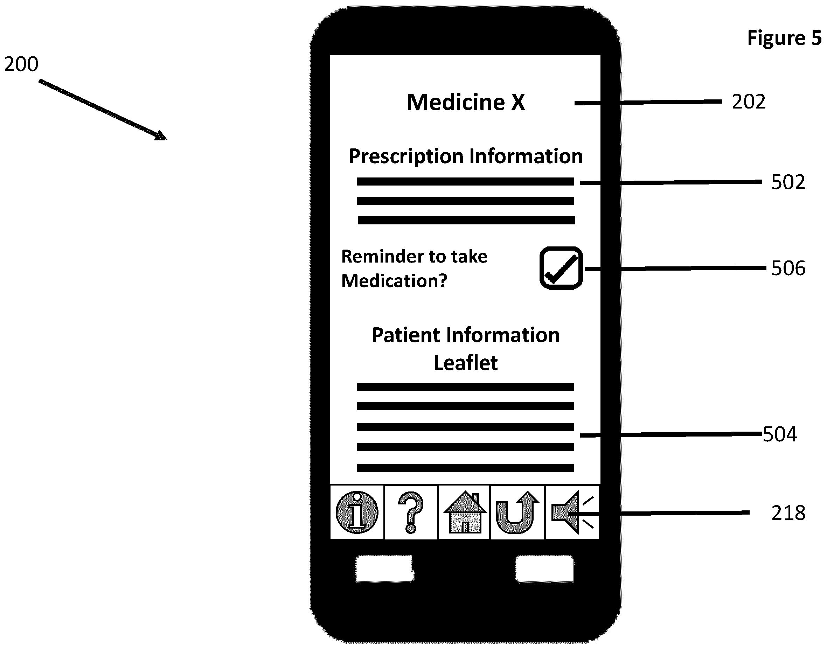

Once the code has been scanned by the mobile terminal 200, the mobile terminal 200 determines an identification associated with the medication based upon the scanned code. This may be a numerical identification or ID number that uniquely identifies the specific packet of medicine that the user scanned with the mobile terminal. The mobile terminal then sends the determined identification to a server and in return receives medicine information associated with the identification from the server. Accordingly, the medicine information is also associated with the medicine in the packet of medicine that was scanned. The medicine information may be presented to the user immediately upon receipt of the medicine information at the mobile terminal following the scanning of the code, and may also be stored locally on the mobile terminal for the user to subsequently access at a later time. Either way, the medicine information may be presented in a manner according to Figure 5.

Figure 5 shows a mobile terminal 200 presenting medicine information on a display 202. The medicine information in this exemplary illustration relates to "Medicine X". The user may be presented with such an application page following their scanning of a packet of "Medicine X" as described in relation to Figure 4 or, if they had previously scanned the packet of "Medicine X", through a list of previously scanned medications illustrated in Figure 3, for example by pressing "Medicine X" button 302.

As can be seen, in the example shown in Figure 5, the medicine information comprises prescription information 502 and an e-PIL 504.

The prescription information 502 comprises information regarding a prescription that the user has received from a doctor or other care giver or health care professional. Typically, this will include information on when to take the medication as well as a prescribed dosage of medication to take. The prescription information 502 is personalised to the user (though is not necessarily unique to them). It may be input to the application through the mobile terminal by the user of the terminal (i.e. a patient to whom the prescription relates), or through the mobile terminal by the person who prescribed the medication to the patient, such as a doctor or other care giver.

In this case, the prescription information 502 may be uploaded to the server by the doctor or other care giver with an identifier that allows the server to determine to which mobile terminal 200 the prescription information 502 should be sent. This may be user account details, such as an account ID number, or through the use of a single use code that the doctor inputs when uploading the patient information to the server and that the patient inputs to the mobile terminal 200 which is then sent to the server with the identification associated with the scanned code. In such a manner, the server can identify the mobile terminal 200 that the prescription information 502 should be sent to and include this in the medicine information along with the generic information that is not personalised to a patient, such as the e-PIL. It is noted that such methods can be used with other types of personalised information as well as the prescription information 502 described above.

The e-PIL 504 comprises an electronic version of the Patient Information Leaflet (PIL) that is included with the medication. The contents of the PIL may vary country by country, and so the e-PIL presented to the user may be based upon a location. This could be a location input manually by the user, or determined by the mobile terminal 200, such as through the use of GNSS (e.g. GPS), WiFi, or a mobile network for example.

In addition to the prescription information 502 and the e-PIL, the medicine information may also comprise further information that may be of relevance to a patient taking that particular medication. The medicine information may be further tailored based on the condition suffered by the patient, which may be input by the patient or received from a doctor or other care giver. This may include advice related to exercise or physical therapies, diet and nutrition.

The medicine information in Figure 5 may also be presented audibly as well as visually or graphically. For example, the user may press the audio button 218 presented on display 202, causing the mobile terminal 200 to read aloud the medicine information. In some cases, a user may configure the application and/or their mobile terminal 200 such that the medicine information is presented audibly automatically or by default. This can overcome problems related to patients lacking the literary skills to read information presented in writing, overcoming a significant drawback of traditional paper PILs.

Additionally, the medicine information may be presented graphically. This could be in the form of a digital avatar. The appearance, voice and language of the digital avatar may vary by location or user settings. A digital avatar can be reassuring for patients and encourage patient compliance with their course of treatment.

Also illustrated in Figure 5 is an option for the user to set notifications or reminders to remind them to take their medication by selecting reminder button 506. If this option is selected, the reminders may be automatically generated based upon the prescription information 502 or a user may be guided through a process of creating one or more reminders. For example, they may cause the mobile terminal 200 to vibrate or emit an audible tone when the prescription information 502 indicates that a patient is due to take some medication. The time, nature and content of a reminder may be customised by a user of the mobile terminal 200. For example, a user may set the reminder such that they receive an alert 5 minutes before they are due to take their medication. Additionally, a user may set multiple reminders, for example alerting them 1 hour before they are due to take their medication as well as 5 minutes before. The reminders may also comprise further information, set by the user or determined from the prescription information 502, such as to remind a patient to ensure they have eaten food before taking their medication.

The reminder may also comprise the option for the patient to confirm when they have taken their medication. Alternatively, this function may be provided elsewhere in the application on the mobile terminal 200. By requesting a patient confirm whether they have taken their medication in this way, patient compliance with the prescribed treatment can be assessed. At present, a doctor or other care giver usually has to rely on a patient's memory as to how well they kept to the prescribed treatment. This is often flawed, and many patients are prone to overestimating how well they kept to the prescribed treatment or may not wish to admit that they have unsuccessfully followed their course of treatment. Accordingly, obtaining compliance data on the go in the above manner can help to overcome these problems and provide the doctor or other care giver an accurate view of the patient's compliance with the prescription.

It will be appreciated that the specific arrangement of buttons and options illustrated in Figures 2 to 5, and the layout and architecture of an application to facilitate a mobile terminal to perform the methods of the invention, are not limited to the examples described, and that any suitable application, software, hardware, or combination thereof may be used that facilitates the implementation of the methods of the invention.

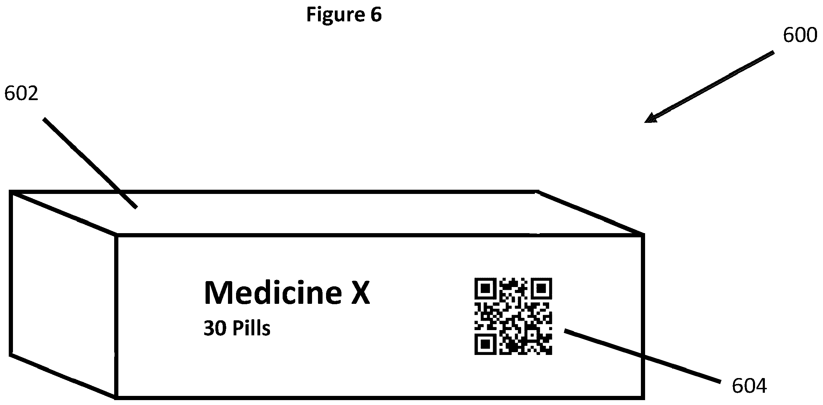

Figure 6 illustrates a medication packet 600 compatible with the present invention. Medication packet 600 comprises packaging, in this case box 602, with a code 604 disposed upon the box 602 (though this need not necessarily be on the surface of the box 602 depending upon how the code 604 is to be scanned). Preferably, the code 604 is in a form that can be readily scanned by mobile phones or other types of mobile terminal, such as tablets or laptop computers. Codes may be scanned or identified optically, or through another means such as Bluetooth, Near Field Communication (NFC), and Radio Frequency Identification (RFID). Suitable codes include barcodes, such as linear or one dimensional barcodes, or two dimension or matrix barcodes. Suitable examples include QR codes and a GS 1 2D DataMatrix.

The code 604 encodes an identifier which is uniquely associated with the specific medication packet 600 on which the code is disposed. By scanning the code 604, a mobile terminal can determine the identifier associated with the code, and send this to a server to obtain the medicine information associated with the medicine. In one embodiment, the identifier associated with the code may be a Global Trade Number. Such codes are already present on medicine packets and as such would allow the method of the invention to be implemented with existing medication packaging.

A code 604 may be associated with a whole packet of medicine, as in the case of Figure 6 in which code 604 is associated with the box 602 which contains 30 individual pills. Alternatively, or in addition, to a code 604 being associated with a packet of medication 600 containing a plurality of individual pills, capsules or other measure of medication, a code may be associated with an individual pill, capsule, or other measure of medication.

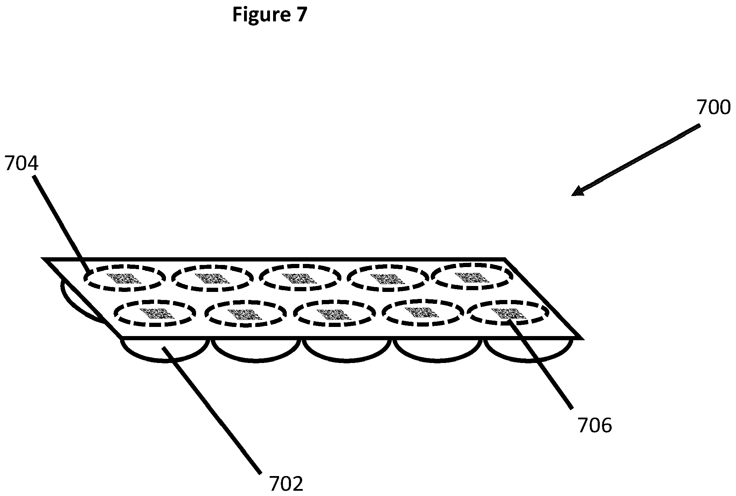

For example, Figure 7 shows a blister pack 700 comprising 10 individual pills. For example, medication packet 600 may comprise three such blister packs 700. The pills are disposed in individual compartments 702 in a plastic tray and covered with a layer of foil. A region of the foil 704 is disposed above each individual compartment 702 and retains the pill in the compartment 702. On top of each of these regions 704, a code 706 may be disposed. Unlike code 604 illustrated in Figure 6 which is associated with a packet of medication containing a plurality of pills, each code 706 is only associated with a single pill.

By providing a code 706 on the blister pack 700 for each individual compartment 702, a patient can access medicine information (such as an e-PIL) even if they have only been given a portion of the blister pack 700, rather than being provided with the whole medication packet 600. Such situations frequently occur in developing countries, when people cannot afford to buy a whole packet of medication 600 and so buy the medication they need for a given day each morning. In this situation, a doctor or pharmacist may cut out a given number of individual compartments 702 from a blister pack 700 to provide to the patient. The present invention, therefore, provides a method by which the problem of ensuring everyone who takes a medication has access to the relevant information regarding that medication, particularly the PIL.

In addition to providing an e-PIL and prescription information, the medicine information may also contain authentication information. The authentication information can provide information to the patient regarding whether the medicine that they scanned is authentic.

When the server receives the identification based on the scanned code from the mobile terminal, it may authenticate the identification. This may involve comparing the identification to a list of authentic identifications stored on the server or a database to determine if the identification received from the mobile terminal is present in the list of authentic identifications. If the authentication is successful then the medicine information provided by the server may comprise an indication that the identification was successfully authenticated. If the authentication was not successful, then the medicine information provided by the server may comprise an indicator that the identification was not authenticated. This can reassure the patient that they have received genuine medication, or alert them to the fact that they have received counterfeit medication, according to the indication in the medicine information.

The server may also perform a number of other checks to help validate the authenticity of the medicine. In one embodiment, the server may determine whether the identification code received from the mobile terminal has already been authenticated previously. That is, the server can determine whether it has previously been asked to authenticate the same identification. If the identification is an authentic identification, but the server has previously authenticated the identification, then this could be an indicator that the medicine is counterfeit but re-using authentic packaging. Accordingly, if this is the case, the medicine information can comprise an indicator that the medicine may not be authentic.

Because a patient may scan the same packaging more than once, for example if they forget that they have already scanned the medicine packet and so scan it a second time, the server may only provide an indication that the medicine may not be authentic if it previously received and authenticated the identification from a different mobile terminal, or from a different user account.

The server may also be able to identify when it receives an identification from a patient application or from a distributor. For example, the medicine may also be scanned at various points in the supply network, such as when leaving a factory, when received by a distributor, and when delivered to a pharmacy. When scanned at these points, the server may also receive the identification which allows it to log a distribution history of the medicine associated with the identification. The server is able to distinguish when the identification is received from a distributor or from a mobile terminal associated with a patient, and only produce a warning that the medication may not be authentic if it has previously received the identification from another patient mobile terminal rather than from a distributor.

The server can also compare the last known location of the medication, based upon the determined distribution history, with a location of the mobile terminal of the patient that scanned the code and sent the identification to the server. If these locations differ in a predetermined manner, or by a predetermined distance, the server may include an alert for the patient in the medicine information. For example, such a disagreement between a previous location in the distribution history and the current location of the mobile terminal may indicate that the medicine was stolen.

When individual pills, or another unit of medication, each have a code associated with them as well as a packet of medication having an associated code, as described with relation to Figure 7, the code associated with each pill may be unique. This would allow each individual pill to be tracked and authenticated in the above manner. Alternatively, codes associated with the individual pills may be a generic code associated with that type of medication, or may be the same as the code associated with the packet of medication that the pills are sold in. This reduces the complexity of the system by greatly reducing the number of unique codes required, but means that individual pills cannot be tracked and authenticated when separated from the packet of medication that does have its own unique code.

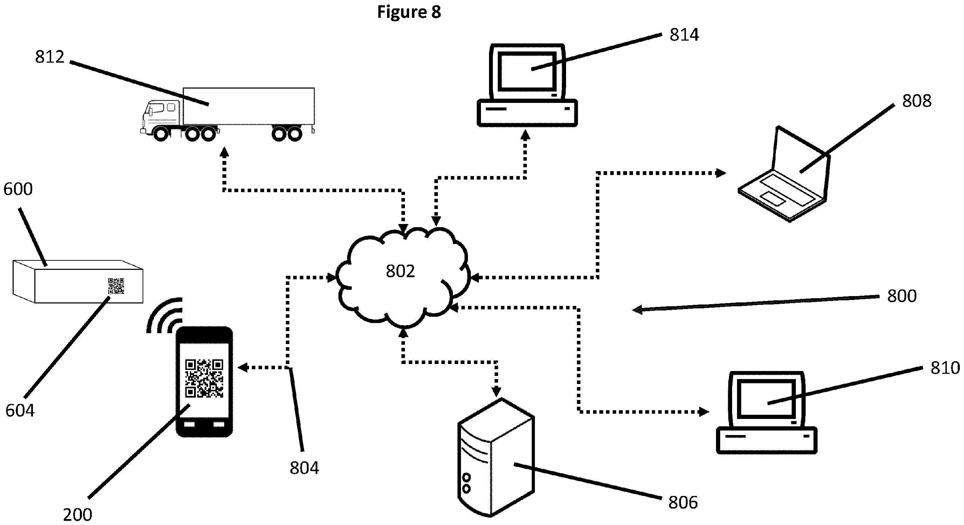

Figure 8 illustrates an exemplary system with which the invention can be used.

As can be seen, a packet of medication 600 is provided with a code 604 which is scanned by mobile terminal 200. Mobile terminal 200 is connected to a network 802 via connection 804. Via the network 802, the mobile terminal 200 can communicate with server 806. This allows the mobile terminal 200 to send an identification, determined based on the scanned code 604, to the server 806. Based on the identification, the server 806 can obtain medicine information corresponding to the medicine in the packaging 600 that was scanned. This may be done by querying a database (not shown), which may be external to the server. When the server 806 has obtained the medicine information, the server 806 sends the medicine information back to the terminal 200 via the network 802 for presentation to the patient.

The server 806 and/or the database (not shown) may store the medicine information in multiple languages, and store different medicine information for sending to mobile terminals 200 in different countries. The mobile terminal 200 may send the server 806 an indication of its location, and the server 806 may send the mobile terminal 200 the appropriate medicine information based upon the location of the mobile terminal 200. Alternatively, or in addition, the server 806 may send the mobile terminal 200 medicine information in part based upon user settings at the mobile terminal 200, such as language settings. For example, the server 806 may send the medicine information in the same language that the mobile terminal 200 is configured to operate it.

The communication of the mobile terminal 200 with the server 806, and the server 806 itself and any associated databases, may be secure and/or encrypted. This may be done with any suitable methods known in the art.

Other devices may also be in communication with the mobile terminal 200 and/or the server 806. For example, a terminal 810 associated with a doctor or other care giver may also be connected to network 802. The doctor or other care giver may be the person who prescribed the medicine. Through their terminal 810, the doctor or other care giver may upload prescription information for the patient to the mobile terminal 200 or the server 806, from which the mobile terminal 200 can obtain it. Having the mobile terminal 200 obtain the prescription information from the prescriber of the medication (rather than relying on the patient to input it) can be advantageous as it reduces the risk of the prescription information being erroneously input.

As well as the doctor or other care giver providing the mobile terminal 200 with the prescription information via terminal 810, the mobile terminal 200 can also communicate patient compliance information to the doctor or other care giver's terminal 810. This can be done automatically each time the patient indicates that they have taken their medication, or in one or more batches. This means that a doctor or other care giver can easily view patient compliance information from their terminal 810, which can in turn enable them to provide more tailored care to the patient to improve patient outcomes.

A terminal 808 associated with a pharmacy may also be connected to network 802. This can allow a patient to renew or re-order their prescription and order new medication through the mobile terminal 200. In some embodiments, the mobile terminal 200 may determine when the patient is due to run out of medication based upon the medicine information. For example, the medicine information may indicate that the patient scanned a pack of 30 pills and that the patient is supposed to take three pills per day. Accordingly, the mobile terminal 200 may determine that the patient will run out of medication after 10 days. The mobile terminal 200 may also take into account the amount of medication that the patient has indicated they have taken. The mobile terminal 200 may provide the patient with a notification to alert them when they are due to run out of medication, such as the day before they are due to run out of medication, and a link to an online pharmacy service to re-order their medication. In some embodiments, the mobile terminal 200 may automatically re-order medication from the pharmacy by communicating with terminal 808 via the network 802 when it is determined that the patient is due to run out of medication

Suppliers and distributors 812 of the medication may also each be connected to the network 802. This may be through respective terminal devices such as terminal 814, which is able to provide data to the server 806 via the network 802. This may be over network 802, or a different network, and this functionality may be provided to the suppliers and distributors using known means such as an application running on a user terminal device such as a mobile phone, tablet or similar. The code 604 may be scanned at various predetermined points in the supply chain of the medication, such as when medicine arrives at or leaves a warehouse, wholesaler or pharmacist, among other points in the supply chain. Based on this, the server 806 can generate a distribution history of the packet of medicine 600, which can be stored in a database accessible to the server 806.

Distributors 812 may not scan every code 604 on every packet 600 of medicine that they transport or handle. Instead, codes may be aggregated. In this case, a single code can be associated with multiple packets of medicines 600 each having their own individual codes 604. This allows a group of packets to easily be tracked. Another example of such aggregation is a medicine packet having a code disposed thereon, with each individual pill inside also having an associated code, as described with relation to Figure 7. When an aggregate code, associated with a plurality of codes that relate to a smaller unit of medicine, is scanned, the server 806 may update a status (such as a distribution event) of each of the codes associated with the aggregate code in a database. In this manner, multiple packets of medication can be tracked and have a distribution history created based on the scanning of a small number of aggregate codes, rather than requiring each packet of medication to have its code individually scanned throughout the supply chain.

By implementing the above described features in any appropriate combination embodiments of the invention may provide a simple to scan medication pack and an intuitive simple to use application (or similar software) with customised content. The medication pack can be scanned to authenticate and ensure the patient has genuine medicine digitally without the need of embedding the packaging design with complex covert printed security features. This also allows the product to be tracked forward throughout the supply chain and traced back from the market to its origin point. In addition, the ability to combine a myriad of different digital features and benefits triggered from the medication packaging is advantageous.

## Claims

1. A method of providing medicine information to a patient, the method comprising:
scanning, at a mobile terminal associated with the patient, a code disposed upon packaging for a medication;
determining, by the mobile terminal, an identification associated with the medication based upon the scanned code;
sending, from the mobile terminal to a server, the determined identification;
receiving, at the mobile terminal from the server, medicine information associated with the identification; and
presenting, at the mobile terminal, the medicine information to the patient.

2. The method of claim 1, wherein the medicine information comprises a Patient Information Leaflet (PIL).

3. The method of claim 1 or 2, further comprising the step of:
authenticating, by the server, the identification;
wherein if the server is able to authenticate the identification then the medicine information comprises an indicator that the identification was authenticated, and if the server is not able to authenticate the identification then the medicine information comprises an indicator that the identification was not authenticated.

4. The method of claim 3, further comprising the step of:
determining, by the server, if the server has previously authenticated the identification;
wherein if the server is able to authenticate the identification but has previously authenticated the same identification then the medicine information comprises an indicator that the medicine may not be authentic.

5. The method of claim 3 or 4, further comprising the steps of:
querying, by the server, a database to obtain a distribution history of the medication based on the identification;
receiving, at the server from the database, the distribution history of the medication based on the identification;
wherein if the server is able to authenticate the identification then the medicine information further comprises a distribution history of the medication.

6. The method of any preceding claim, wherein prior to sending the determined identification to the server, the method further comprises, at the mobile terminal:
determining the location of the mobile terminal;
wherein the step of sending the determined identification further comprises sending, to the server, the location of the mobile terminal; and
wherein the medicine information received from the server is at least in part based upon the location of the mobile terminal.

7. The method of any preceding claim, wherein the method further comprises the steps of, at the mobile terminal:
receiving prescription information, the prescription information specifying one or more times at which the patient should take the medication and a dosage of the medication to be taken at the one or more times;
receiving patient confirmation when the patient has taken a dose of the medication; and
determining patient compliance with the prescription information,
wherein optionally the method further comprises the step of:
sending, from the mobile terminal to a patient's care provider, information relating to the determined patient compliance.

8. The method of claim 7, wherein the medicine information comprises an initial amount of medication associated with the identification, and the method further comprises the steps of, at the mobile terminal:
updating the amount of medication associated with the identification by decreasing the amount based upon the prescription information and/or determined patient compliance;
notifying the patient when the updated amount of medication passes a predetermined threshold that indicates that the medication has almost been consumed;
wherein optionally the method further comprises the step of:
automatically ordering new medication, by the mobile terminal, when the updated amount of medication passes the predetermined threshold.

9. The method of any preceding claim, wherein the medicine information further comprises a link to a page of an online pharmacy through which the patient can order new medication.

10. The method of any of claims 7 to 9, wherein the prescription information is received from a terminal associated with a health care provider, wherein optionally the healthcare provider is one of a doctor, a pharmacist, or the prescriber of the medication.

11. The method of any of claims 7 to 10, wherein the method further comprises the step of:
outputting a notification to alert the patient when the prescription information indicates that the patient is due to take some or all of the medication;
wherein optionally the notification to alert the patient when the prescription information indicates that the patient is due to take some or all of the medication is output at a time based on at least the prescription information and user input from the patient.

12. The method of any preceding claim, wherein the code is associated with an individual pill or capsule; or wherein the code is associated with a packet comprising multiple individual pills or capsules, and wherein the code can further identify each constituent pill or capsule in the packet.

13. The method of any preceding claim, wherein at least a part of the medicine information is presented audibly or graphically, wherein optionally at least a part of the medicine information is presented through a digital avatar.

14. A system comprising a mobile terminal and a server, configured to perform the method of any preceding claim.

15. A mobile terminal or a server, configured for use in the system of claim 14.
